(19) Europäisches Patentamt
European Patent Office
Office européen des brevets

(11) Numéro de publication : **0 283 411 B1**

(12) # FASCICULE DE BREVET EUROPEEN

(45) Date de publication du fascicule du brevet : **07.08.91 Bulletin 91/32**

(51) Int. Cl.[5] : **C07C 37/62**, C07C 39/32, C07C 39/28

(21) Numéro de dépôt : **88420078.3**

(22) Date de dépôt : **03.03.88**

(54) **Procédé de chloration de composés phénoliques.**

(30) Priorité : **05.03.87 FR 8703208**

(43) Date de publication de la demande : **21.09.88 Bulletin 88/38**

(45) Mention de la délivrance du brevet : **07.08.91 Bulletin 91/32**

(84) Etats contractants désignés : **AT BE CH DE ES GB GR IT LI LU NL SE**

(56) Documents cités :
**EP-A- 0 002 373**
**EP-A-01 962 60**
**GB-A- 573 477**
**GB-A- 2 177 396**

(73) Titulaire : **RHONE-POULENC CHIMIE**
**25, quai Paul Doumer**
**F-92408 Courbevoie Cédex (FR)**

(72) Inventeur : **Besson, Bernard**
**15, rue de Moucherotte**
**F-38800 Pont de Claix (FR)**
Inventeur : **Desmurs, Jean-Roger**
**La Jonquière Route de Ternay Communay**
**F-69360 St-Symphorien d'Ozon (FR)**
Inventeur : **Jouve, Isabelle**
**30, rue Léon Fabre**
**F-69100 Villeurbanne (FR)**

(74) Mandataire : **Vignally, Noel et al**
**Rhône-Poulenc Interservices Service Brevets**
**Chimie Centre de Recherches des Carrières**
**B.P. 62**
**F-69192 Saint-Fons Cédex (FR)**

EP 0 283 411 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de chloration en position para par rapport à la fonction OH par le chlore gazeux de composés phénoliques substitués en positions ortho par rapport à la fonction hydroxyle.

Parmi les composés phénoliques importants, qui sont susceptibles d'être obtenus par chloration d'un composé phénolique substitué en positions ortho, se trouve le trichloro-2,4,6 phénol.

Le procédé habituel de préparation du trichloro-2,4,6 phénol consiste dans la chloration du dichloro-2,4 phénol.

Cependant il se forme une faible proportion de trichloro-2,4,5 phénol (de l'ordre de 0,003 à 0,010% du poids de trichloro-2,4,6 phénol). Le trichloro-2,4,6 phénol, qui est un intermédiaire pour la synthèse d'autres composés, ne doit pas contenir de traces de cet isomère indésirable.

Dans EP-A 0196260, on a décrit un procédé de chloration sélective des composés phénoliques par le chlore gazeux qui met en oeuvre une amine. Toutefois, ce document ne décrit qu'une chloration sélective en position ortho par rapport à la fonction hydroxyle des composés phénoliques.

Une solution à ce problème d'obtention de trichloro-2,4,6 phénol,consisterait donc à chlorer le dichloro-2,6 phénol, ce qui éviterait totalement la formation de trichloro-2,4,5 phénol. Le trichloro-2,3,6 phénol, susceptible de se former dans ce cas, à l'état de traces, est beaucoup moins gênant que le trichloro-2,4,5 phénol.

En fait, lorsque l'on chlore par le chlore gazeux le dichloro-2,6 phénol, on constate que l'on n'obtient pas un excellent rendement. Il se forme notamment une quantité importante de pentachloro-2,4,5,6,6 cyclohexène-2 one, ce qui rend le mélange réactionnel très instable et difficile à purifier.

La présente invention se propose de résoudre ce problème, ainsi que le problème plus général de la chloration en position para, avec un bon rendement, des composés phénoliques ayant des substituants en positions ortho par rapport à la fonction hydroxyle.

Plus précisément, elle concerne un procédé de chloration en position para par rapport à la fonction OH par le chlore gazeux de composés phénoliques de formule générale (I) :

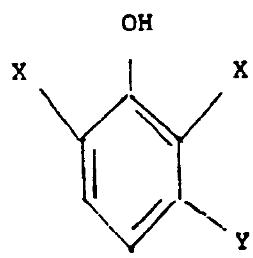

(I)

dans laquelle :

— les symboles X, identiques ou différents, représentent un atome de chlore, un atome de brome, un groupement méthyle ou éthyle, un groupement méthoxy ou éthoxy, un groupement acétoxy, un groupement $NO_2$, un groupement acylamino ayant 1 à 4 atomes de carbone,

— le symbole Y représente un atome d'hydrogène, un groupement méthyle ou éthyle, un groupement méthoxy ou éthoxy,

caractérisé en ce que l'on opère en présence d'une quantité efficace d'au moins une amine primaire, secondaire ou tertiaire.

Par amine, on entend dans le présent texte tout composé, liquide ou solide dans les conditions opératoires du procédé, comportant une ou plusieurs fonctions amine.

Un tel composé peut également comporter une ou plusieurs autres fonctions chimiques, telles que par exemple la fonction hydroxyle, la fonction acide carboxylique, la fonction ester d'acide carboxylique, la fonction amide ou la fonction imine.

Il est bien évident que les amines utilisées peuvent être introduites également sous la forme de leurs sels et plus particulièrement de leurs chlorhydrates respectifs.

Dans le présent texte, le terme "amine" englobe également l'ammoniac ainsi que les sels, notamment les chlorhydrates d'amines.

Le procédé selon l'invention peut être conduit en l'absence de solvant : les réactifs sont alors à l'état fondu. Cette variante de l'invention conduit généralement aux meilleurs résultats.

On peut également opérer dans un milieu liquide constitué notamment par un éther aliphatique, un hydrocarbure aliphatique, un hydrocarbure aliphatique chloré, un chlorobenzène ou un bromobenzène.

A titre d'éther aliphatique, on peut citer notamment l'oxyde de dipropyle, l'oxyde de diisopropyle, l'oxyde

de méthyle et de tertiobutyle.

A titre d'hydrocarbure aliphatique, on peut citer notamment l'hexane, l'heptane, l'octane, le nonane et le décane.

A titre d'hydrocarbure chloré, on peut citer les hydrocarbures perchlorés tels que notamment le tétrachlorure de carbone, le tétrachloroéthylène, l'hexachloroéthane, l'hexachloropropène et l'hexachlorobutadiène ; les hydrocarbures partiellement chlorés tels que le chlorure de méthylène, le dichloroéthane, le tétrachloroéthane, le trichloroéthylène, le chloro-1 butane, le dichloro-1,2 butane.

A titre de chlorobenzène, on peut citer en particulier le monochlorobenzène, le dichloro-1,2 benzène, le dichloro-1,3 benzène, le dichloro-1,4 benzène ou des mélanges de différents chlorobenzènes ; à titre de bromobenzène, on peut citer notamment le monobromobenzène ou des mélanges de monobromobenzène avec un ou plusieurs dibromobenzènes.

Lorsque le procédé de l'invention est mis en oeuvre en milieu solvant, la concentration du chlorophénol de formule (I) dans le solvant n'est pas critique. Elle dépendra notamment de la solubilité du chlorophénol dans le solvant utilisé.

Les amines utilisées comme catalyseur dans le présent procédé sont plus particulièrement les amines de formule générale (II) :

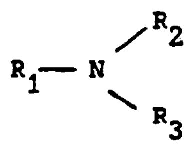

(II)

dans laquelle :

— $R_1$, $R_2$ et $R_3$, identiques ou différents, représentent :

- un radical alkyle linéaire ayant de 1 à 12 atomes de carbone, un radical alkyle secondaire ayant 3 à 12 atomes de carbone ou un radical alkyle tertiaire ayant 4 à 12 atomes de carbone, ces radicaux alkyles pouvant comporter une ou deux fonctions éther —O— ou fonctions hydroxyle, amine, acide carboxylique, ester d'acide carboxylique, amide ou imine ;
- un radical phényle, un radical cyclohexyle, un radical cycloheptyle ou un radical cyclopentyle ;
- un radical phénylalkyle, cyclohexylalkyle, cycloheptylalkyle ou cyclopentylalkyle dont la partie alkyle comporte 1 à 4 atomes de carbone ;
- un atome d'hydrogène.

— $R_1$ peut représenter un groupement $NH_2$ ;

— $R_2$ et $R_3$ forment ensemble et avec l'atome d'azote un hétérocycle, saturé ou comportant une ou plusieurs doubles liaisons, éventuellement substitué par un ou plusieurs groupements alkyles ayant 1 à 4 atomes de carbone ;

— $R_2$ et $R_3$ ou $R_1$, $R_2$ et $R_3$ forment ensemble, avec l'atome d'azote et avec un ou plusieurs atomes d'azote et/ou d'oxygène et/ou de soufre, un hétérocycle, saturé ou insaturé, éventuellement substitué par un ou plusieurs groupements alkyles ayant 1 à 4 atomes de carbone ;

— $R_1$, $R_2$ et $R_3$ forment ensemble et avec l'atome d'azote un hétérocycle insaturé éventuellement substitué par un ou deux groupements méthyle ou éthyle ;

— $R_2$ et $R_3$ ou $R_1$, $R_2$ et $R_3$ forment ensemble, avec l'atome d'azote et éventuellement avec un ou plusieurs atomes d'azote et/ou d'oxygène et/ou de soufre, un composé polycyclique, saturé ou insaturé, éventuellement substitué par un ou plusieurs groupements alkyles ayant 1 à 4 atomes de carbone.

A titre d'exemples illustratifs, on peut citer comme amines de formule (II) :

— l'ammoniac ;

— des amines primaires telles que la n-propylamine, l'isopropylamine, l'isobutylamine, la n-butylamine, la tertiobutylamine, la n-pentylamine, la méthyl-2 butylamine, la méthyl-3 butylamine, la n-hexylamine, l'éthyl-2 hexylamine, l'aniline, la laurylamine, la cyclohexylamine, la cyclopentylamine, la benzylamine, la guanidine, l'acétamidine, l'ester éthylique de la glycine, l'éthanolamine, l'éthylènediamine, l'hexaméthylènediamine, la N-aminoéthylpyrrolidine, la pyrazoline, la lysine, la N-aminomorpholine, la N-aminopipéridine ;

— des amines secondaires telles que la dibutylamine, la dipropylamine, la méthylpropylamine, la méthylbutylamine, la méthylisobutylamine, la méthyltertiobutylamine, la méthylbenzylamine, la ditertiobutylamine, la méthyl-1 cyclopentylamine, la méthyl-1 cyclohexylamine, la dicyclohexylamine, la morpholine, l'imidazole, la pyrrolidine, l'imidazolidine, la pipérazine, l'indole ;

— des amines tertiaires telles que la triéthylamine, la tributylamine, la pyridine, la tris (dioxa-3,6 heptyl) amine, le diaza-1,8 bicyclo(5,4,0)undécène-7.

On peut utiliser également des composés aminés comme l'hydrazine ou ses dérivés, notamment les dérivés obtenus par substitution d'un ou de deux atomes d'hydrogène par des radicaux alkyles, aryles, cycloaliphatiques ou hétérocycliques.

La quantité d'amine utilisée dans le procédé peut varier dans de très larges limites.

Habituellement elle représente en poids par rapport au poids du composé phénolique de formule (I) de 0,005% à 25%.

Lorsque l'on opère à l'état fondu, on mettra en oeuvre préférentiellement de 0,015% à 5% en poids d'amine par rapport au composé phénolique de formule (I), afin d'avoir une efficacité suffisante, tout en n'ayant pas une quantité excessive d'amine.

Lorsque l'on opère en milieu solvant, on mettra en oeuvre préférentiellement de 5 à 25% en poids d'amine par rapport au composé phénolique de formule (I). Dans ce cas il est préférable d'exprimer la quantité d'amine par rapport au mélange réactionnel. On utilisera par exemple de 0,01% à 2% en poids d'amine par rapport au mélange réactionnel.

Parmi les amines de formule générale (II), on préfère dans le cadre du présent procédé, les amines primaires ou secondaires de formule (III) :

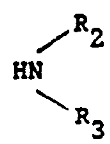 (III)

dans laquelle :

— $R_2$ ou $R_3$ représente un atome d'hydrogène ;

— $R_2$ et $R_3$, identiques ou différents, représentent :

- un radical alkyle linéaire ayant 1 à 10 atomes de carbone ;
- un radical alkyle secondaire ayant 3 à 10 atomes de carbone ;
- un radical alkyle tertiaire ayant 4 à 10 atomes de carbone ;
- un radical cyclohexyle ou cyclopentyle ;
- un radical phényle ;
- un radical benzyle ou phénéthyle ;

— $R_2$ et $R_3$ forment ensemble, avec l'atome d'azote et avec un autre atome d'azote et/ou d'oxygène, un hétérocycle saturé ou comportant une ou plusieurs insaturations ;

— $R_2$ et/ou $R_3$ comportent une ou plusieurs fonctions amine, hydroxyle ou ester d'acide carboxylique.

A titre d'exemples d'amines primaires de formule générale (III), on peut citer la n-propylamine, l'isopropylamine, la n-butylamine, l'isobutylamine, la tertiobutylamine, la n-pentylamine, la méthyl-2 pentylamine, la méthyl-3 pentylamine, l'éthyl-2 hexylamine, la laurylamine, la cyclohexylamine, la cyclopentylamine, la benzylamine, l'ester éthylique de la glycine et l'éthanolamine.

En ce qui concerne plus particulièrement les amines secondaires de formule générale (III), on préfère encore plus spécialement celles pour lesquelles au moins un des symboles $R_2$ et $R_3$ et de préférence les deux symboles $R_2$ et $R_3$ représentent :

- un radical alkyle secondaire ayant de 3 à 10 atomes de carbone tel qu'isopropyle, butyle-2, pentyle-2, pentyle-3, hexyle-2, hexyle-3, heptyle-2, heptyle-3, heptyle-4, octyle-2, octyle-3, octyle-4, nonyle-2, nonyle-3, nonyle-4, nonyle-5, décyle-2, décyle-3, décyle-4 et décyle-5 ;
- un radical cyclohexyle ou cyclopentyle ;

et celles pour lesquelles $R_2$ et $R_3$ forment avec l'atome d'azote un hétérocycle comportant éventuellement un autre atome d'azote ou un atome d'oxygène.

A titre d'exemples de telles amines secondaires, on peut citer la diisopropylamine, la diisobutylamine, la dicyclohexylamine, la morpholine, l'imidazole.

La quantité de chlore utilisée dans le procédé selon l'invention est essentiellement fonction du taux de transformation souhaité du composé phénolique (I).

En pratique, le plus souvent, le chlore est introduit par barbotage dans le milieu réactionnel. La pression dans l'appareillage est donc sensiblement égale ou légèrement supérieure à la pression atmosphérique.

Le chlore peut être utilisé seul ou être dilué par un gaz inerte, tel que l'azote par exemple. La présence d'un gaz inerte permet, si nécessaire, d'augmenter le débit gazeux sans augmenter corrélativement la quantité

de chlore introduite en un temps donné.

Le chlore gazeux utilisé dans le présent procédé peut également être formé in situ, à partir d'acide chlorhydrique, par addition d'un composé oxydant, tel que par exemple le peroxyde d'hydrogène.

La température à laquelle est mis en oeuvre le procédé de l'invention est généralement inférieure ou égale à 180°C. La limite inférieure n'est pas critique. Elle est conditionnée par la nécessité d'avoir un mélange réactionnel liquide.

Lorsque l'on opère à l'état fondu, cette température inférieure variera donc selon le composé phénolique (I) soumis à la chloration. Ainsi lorsque l'on chlore le dichloro-2,6 phénol, il faudra une température d'au moins 65°C.

Lorsque l'on opère en milieu solvant, on pourra descendre la température jusqu'à 20°C par exemple.

De préférence cependant, la température sera comprise entre 40°C et 120°C, si l'on opère en milieu solvant.

Si l'on opère à l'état fondu, les températures préférées seront comprises entre 40 et 120°C, sauf bien entendu pour les composés phénoliques ayant un point de fusion supérieur à 40°C, pour lesquels la zone préférée de température sera comprise entre leur point de fusion et 120°C.

Parmi les composés phénoliques de formule (I) auxquels peut s'appliquer le procédé de l'invention, on peut citer plus particulièrement le dichloro-2,6 phénol, le diméthoxy-2,6 phénol, le chloro-2 méthoxy-6 phénol, le chloro-2 méthyl-6 phénol, le dichloro-2,6 méthyl-3 phénol, le dichloro-2,6 méthoxy-3 phénol, le bromo-2 méthoxy-6 phénol, le chloro-2 nitro-6 phénol, le chloro-2 acétamido-6 phénol.

On peut, si on le souhaite, chlorer des mélanges de ces composés phénoliques.

Le procédé de l'invention est tout particulièrement adapté à la chloration du dichloro-2,6 phénol en trichloro-2,4,6 phénol, car il permet d'obtenir ce dernier composé en limitant très fortement, généralement à moins de 3% en poids, la formation de produits secondaires indésirables tels que la pentachloro-2,4,5,6,6 cyclohexène-2 one.

Lorsque le procédé de l'invention est appliqué au dichloro-2,6 phénol, celui-ci peut être obtenu notamment par chloration à l'aide de chlore gazeux, de chloro-2 phénol, en présence d'une amine primaire, secondaire ou tertiaire telle que définie précédemment.

On peut donc ainsi obtenir le trichloro-2,4,6 phénol par chloration du chloro-2 phénol par le chlore gazeux en présence d'une amine, qui catalysera tout d'abord la chloration du chloro-2 phénol en dichloro-2,6 phénol, puis la chloration de ce dernier en trichloro-2,4,6 phénol.

On peut également opérer selon la présente invention la chloration du dichloro-2,6 phénol préparé par chloration du phénol à l'aide de chlore gazeux, en présence d'une amine telle que définie précédemment.

Il s'avère que le procédé de l'invention s'applique également aux mélanges bruts de chloration du phénol, qui contiennent du dichloro-2,6 phénol ainsi que du dichloro-2,4 phénol, de l'orthochlorophénol et éventuellement de faibles quantités de parachlorophénol et phénol.

Appliqué à de tels mélanges industriels, le procédé de l'invention permet d'obtenir avec un excellent rendement, pratiquement uniquement du trichloro-2,4,6 phénol. En outre on ne détecte pratiquement plus de trichloro-2,4,5 phénol, qui est un composé indésirable.

Les conditions décrites pour la chloration des composés phénoliques de formule (I) et plus particulièrement du dichloro-2,6 phénol, s'appliquent à la chloration du phénol et/ou du chloro-2 phénol ou des mélanges industriels bruts de chloration du phénol indiqués précédemment.

Les exemples qui suivent illustrent l'invention.

## EXEMPLE 1

Dans un réacteur en verre de 200 cm3, équipé d'un agitateur, d'une tubulure plongeante permettant l'introduction de chlore gazeux, d'un thermomètre et surmonté d'un réfrigérant, on charge :

| | |
|---|---|
| — dichloro-2,6 phénol : | 32,6 g (0,2 mole) |
| — diisopropylamine : | 0,33 g (soit 1% en poids par rapport au dichloro-2,6 phénol). |

Le mélange réactionnel est chauffé sous agitation à 70°C et le chlore gazeux est alors introduit avec un débit de 5 l/h pendant 54 min, ce qui représente une quantité de chlore de 200 mmol.

En fin de réaction on purge l'appareil par un courant d'azote.

On analyse la masse réactionnelle par chromatographie en phase gazeuse (CPG) et par chromatographie liquide haute performance (CLHP).

On obtient les résultats suivants :

- taux de transformation (TT) du dichloro-2,6 phénol : 85,9 %
- rendement (RT) en trichloro-2,4,6 phénol par rapport au dichloro-2,6 phénol transformé : 95 ,9 %
- RT en tétrachloro-2,3,4,6 phénol : 2,0 %.
- Teneur en trichloro-2,4,5 phénol dans le mélange : 0,0004 %

ESSAI COMPARATIF A

L'exemple 1 est répété, mais sans diisopropylamine.
On obtient les résultats suivants :

- TT du dichloro-2,6 phénol : 66,0 %
- RT en trichloro-2,4,6 phénol : 76,0 %
- RT en tétrachloro-2,3,4,6 phénol : 2,0 %.

EXEMPLE 2

Dans l'appareillage décrit pour l'exemple 1, on charge :

— orthochlorophénol : 32,1 g (0,25 mole)
— diisopropylamine : 0,3 g (soit 1% en poids par rapport à l'orthochlorophénol).

Le mélange réactionnel est chauffé à 70°C sous agitation et le chlore est alors introduit avec un débit de 5 l/h pendant 2 h 19 min, ce qui correspond à une quantité de chlore de 517 mmol.
Le mélange réactionnel est dosé par CPG et CLHP.
On obtient les résultats suivants :

- TT de l'orthochlorophénol : 100 %
- RT en trichloro-2,4,6 phénol : 89,0 %
- RT en tétrachloro-2,3,4,6 phénol : 3,0 %
- RT en tétrachloro-2,4,4,6 cyclohexadiène-2,5 one : 0,5 %.

EXEMPLE 3

Dans l'appareillage décrit pour l'exemple 1, on charge :

— phenol : 32,9 g (0,35 mole)
— diisopropylamine : 0,33 g.

Le mélange réactionnel est chauffé à 70°C sous agitation et le chlore est alors introduit avec un débit de 5 l/h pendant 4 h 29 min, ce qui correspond à une quantité de chlore de 1 mole.
Le mélange réactionnel est dosé par CPG et CLHP.
On obtient les résultats suivants :

- TT du phénol : 100 %
- RT en trichloro-2,4,6 phénol : 91,8 %
- RT en dichloro-2,6 phénol : 1,1 %
- RT en tétrachloro-2,3,4,6 phénol : 1,6 %
- RT en tétrachloro-2,4,4,6 cyclohexadiène-2,5 one : 2,5 %.

EXEMPLE 4

Dans l'appareillage décrit pour l'exemple 1, on charge :

— dichloro-2,6 phénol : 32,6 g (0,20 mole)
— diisopropylamine : 0,03 g (soit 0,1% en poids par rapport au dichloro-2,6 phénol).

Le mélange réactionnel est chauffé à 70°C sous agitation et le chlore est alors introduit avec un débit de 5 l/h pendant 53 min, ce qui correspond à une quantité de chlore de 0,2 mole.

Le mélange réactionnel est dosé par CPG et CLHP.

On obtient les résultats suivants :

- TT du dichloro-2,6 phénol : 71 5 %
- RT en trichloro-2,4,6 phénol : 92,5 %
- RT en tétrachloro-2,3,4,6 phénol : 1,1 %

EXEMPLE 5

Dans l'appareillage décrit pour l'exemple 1, on charge 40 g d'un mélange industriel brut de chlorophénols dont la composition pondérale est la suivante :

— orthochlorophénol : 0,22% (0,7 mmol)
— dichloro-2,6 phénol : 23,63% (56 mmol)
— dichloro-2,4 phénol : 42,32% (103,8 mmol)
— trichloro-2,4,6 phénol : 26,3% (53,2 mmol)
— parachlorophénol : 1,35% (4,2 mmol)
— trichloro-2,4,5 phénol : 0,0822%

Après avoir chauffé sous agitation le mélange à 70°C, on introduit en 1 heure 188,75 mmol de chlore, soit un excès de 10% par rapport à la quantité théorique nécessaire pour transformer la totalité des chlorophénols en trichloro-2,4,6 phénol.

Après avoir purgé le réacteur à l'azote, on analyse le mélange réactionnel final (96,2 g) par CPG et CLHP.

Ce mélange contient environ 88% de trichloro-2,4,6 phénol, ce qui correspond à un rendement d'environ 98% par rapport à l'ensemble des chlorophénols engagés.

La teneur en trichloro-2,4,5 phénol est d'environ 0,0004%.

EXEMPLE 6

Dans un réacteur de 1000 cm3, équipé comme décrit dans l'exemple 1, on charge :

— dichloro-2,6 phénol : 32,6 g (0,2 mole)
— tétrachloroéthylène : 612 g
— diisopropylamine : 6,45 g (soit 1% en poids par rapport au mélange réactionnel).

Le mélange réactionnel est chauffé sous agitation à 70°C et à cette température on introduit en 54 min 4,48 litres de chlore (0,2 mole).

En fin de réaction on purge l'appareil à l'azote. On analyse le mélange réactionnel en CPG et CLHP après évaporation du solvant.

On obtient les résultats suivants :

— TT du dichloro-2,6 phénol : 64%
— RT en trichloro-2,4,6 phénol : 79%
— RT en tétrachloro-2,3,4,6 phénol : 4%.

ESSAI COMPARATIF B

Dans un réacteur de 500 cm3 équipé comme décrit dans l'exemple 1, on charge :

— dichloro-2,6 phénol : 24,45 g (0,15 mole)
— tétrachloroéthylène : 254 g.

Le mélange réactionnel est chauffé sous agitation à 75°C et à cette température on introduit en 40 min 3,36 litres de chlore (0,15 mole).

En fin de réaction on purge l'appareil à l'azote. On analyse le mélange réactionnel en CPG et CLHP après évaporation du solvant.

On obtient les résultats suivants :

— TT du dichloro-2,6 phénol : 3%
— RT en trichloro-2,4,6 phénol : 90%

**Revendications**

1. Procédé de chloration en position para par rapport à la fonction OH par le chlore gazeux de composés phénoliques de formule générale (I) :

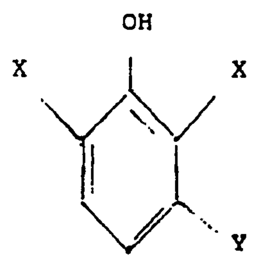

(I)

dans laquelle :

— les symboles X, identiques ou différents, représentent un atome de chlore, un atome de brome, un groupement méthyle ou éthyle, un groupement méthoxy ou éthoxy, un groupement acétoxy, un groupement $NO_2$, un groupement acylamino ayant 1 à 4 atomes de carbone,

— le symbole Y représente un atome d'hydrogène, un groupement méthyle ou éthyle, un groupement méthoxy ou éthoxy,

caractérisé en ce que l'on opère en présence d'une quantité efficace d'au moins une amine primaire, secondaire ou tertiaire.

2. Procédé selon la revendication 1, caractérisé en ce que l'on opère à l'état fondu.

3. Procédé selon la revendication 1, caractérisé en ce que l'on opère dans un solvant.

4. Procédé selon la revendication 3, caractérisé en ce que le solvant est constitué par un éther aliphatique, un hydrocarbure aliphatique, un hydrocarbure aliphatique chloré, un chlorobenzène ou un bromobenzène.

5. Procédé selon l'une des revendications 1 à 4, caractérisé en ce que l'on utilise au moins une amine choisie parmi les amines de formule générale (II) :

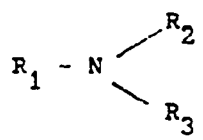 (II)

dans laquelle :

— $R_1$, $R_2$ et $R_3$, identiques ou différents, représentent :

- un radical alkyle linéaire ayant de 1 à 12 atomes de carbone, un radical alkyle secondaire ayant 3 à 12 atomes de carbone ou un radical alkyle tertiaire ayant 4 à 12 atomes de carbone, ces radicaux alkyles pouvant comporter une ou deux fonctions éther —O— ou fonctions hydroxyle, amine, acide carboxylique, ester d'acide carboxylique, amide ou imine ;
- un radical phényle, un radical cyclohexyle, un radical cycloheptyle ou un radical cyclopentyle ;
- un radical phénylalkyle, cyclohexylalkyle, cycloheptylalkyle ou cyclopentylalkyle dont la partie alkyle comporte 1 à 4 atomes de carbone ;
- un atome d'hydrogène.

— $R_1$ peut représenter un groupement $NH_2$ ;

— $R_2$ et $R_3$ forment ensemble et avec l'atome d'azote un hétérocycle, saturé ou comportant une ou plusieurs doubles liaisons, éventuellement substitué par un ou plusieurs groupements alkyles ayant 1 à 4 atomes de carbone ;

— $R_2$ et $R_3$ ou $R_1$, $R_2$ et $R_3$ forment ensemble, avec l'atome d'azote et avec un ou plusieurs atomes d'azote et/ou d'oxygène et/ou de soufre, un hétérocycle, saturé ou insaturé, éventuellement substitué par un ou plusieurs groupements alkyles ayant 1 à 4 atomes de carbone ;

— $R_1$, $R_2$ et $R_3$ forment ensemble et avec l'atome d'azote un hétérocycle insaturé éventuellement substitué par un ou deux groupements méthyle ou éthyle ;

— $R_2$ et $R_3$ ou $R_1$, $R_2$ et $R_3$ forment ensemble, avec l'atome d'azote et éventuellement avec un ou plusieurs atomes d'azote et/ou d'oxygène et/ou de soufre, un composé polycyclique, saturé ou insaturé, éventuellement substitué par un ou plusieurs groupements alkyles ayant 1 à 4 atomes de carbone.

6. Procédé selon l'une des revendications 1 à 5, caractérisé en ce que l'on utilise de 0,005% à 25% en poids d'amine par rapport au composé phénolique de formule (I).

7. Procédé selon la revendication 2, caractérisé en ce que l'on utilise de 0,015% à 5% en poids d'amine par rapport au composé phénolique (I).

8. Procédé selon l'une des revendications 3 ou 4, caractérisé en ce que l'on utilise de 0,01% à 2% en poids d'amine par rapport au mélange réactionnel.

9. Procédé selon l'une des revendications 1 à 8, caractérisé en ce que l'amine utilisée est choisie parmi les amines primaires ou secondaires de formule (III) :

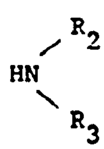 (III)

dans laquelle :

— $R_2$ ou $R_3$ représente un atome d'hydrogène ;

— $R_2$ et $R_3$, identiques ou différents, représentent :

- un radical alkyle linéaire ayant 1 à 10 atomes de carbone ;
- un radical alkyle secondaire ayant 3 à 10 atomes de carbone ;
- un radical alkyle tertiaire ayant 4 à 10 atomes de carbone ;
- un radical cyclohexyle ou cyclopentyle ;
- un radical phényle ;
- un radical benzyle ou phénéthyle ;

— $R_2$ et $R_3$ forment ensemble, avec l'atome d'azote et avec un autre atome d'azote et/ou d'oxygène, un hétérocycle saturé ou comportant une ou plusieurs insaturations ;

— $R_2$ et/ou $R_3$ comportent une ou plusieurs fonctions amine, hydroxyle ou ester d'acide carboxylique.

10. Procédé selon l'une des revendications 1 à 8, caractérisé en ce que l'amine utilisée est choisie parmi les amines secondaires de formule générale (III) dans laquelle au moins un des symboles $R_2$ et $R_3$ et de préférence les deux symboles $R_2$ et $R_3$ représentent :

- un radical alkyle secondaire ayant de 3 à 10 atomes de carbone tel qu'isopropyle, butyle-2, pentyle-2, pentyle-3, hexyle-2, hexyle-3, heptyle-2, heptyle-3, heptyle-4, octyle-2, octyle-3, octyle-4, nonyle-2, nonyle-3, nonyle-4, nonyle-5, décyle-2, décyle-3, décyle-4 et décyle-5 ;
- un radical cyclohexyle ou cyclopentyle ;

et celles pour lesquelles $R_2$ et $R_3$ forment avec l'atome d'azote un hétérocycle comportant éventuellement un autre atome d'azote ou un atome d'oxygène.

11. Procédé selon l'une des revendications 9 ou 10, caractérisé en ce que l'on utilise la diisopropylamine, la diisobutylamine, la dicyclohexylamine, la morpholine et/ou l'imidazole.

12. Procédé selon la revendication 9, caractérisé en ce que l'on utilise la n-propylamine, l'isopropylamine, la n-butylamine, l'isobutylamine, la tertiobutylamine, la n-pentylamine, la méthyl-2 pentylamine, la méthyl-3 pentylamine, l'éthyl-2 hexylamine, la laurylamine, la cyclohexylamine, la cyclopentylamine, la benzylamine, l'ester éthylique de la glycine et/ou l'éthanolamine.

13. Procédé selon l'une des revendications 1 à 12, caractérisé en ce que le composé phénolique de formule (I) est choisi parmi le dichloro-2,6 phénol, le diméthoxy-2,6 phénol, le chloro-2 méthoxy-6 phénol, le chloro-2 méthyl-6 phénol, le dichloro-2,6 méthyl-3 phénol, le dichloro-2,6 méthoxy-3 phénol, le bromo-2 méthoxy-6 phénol, le chloro-2 nitro-6 phénol, le chloro-2 acétamido-6 phénol.

14. Procédé selon l'une des revendications 1 à 12, caractérisé en ce que le composé phénolique de formule (I) est le dichloro-2,6 phénol.

15. Procédé selon l'une des revendications 1 à 14, caractérisé en ce que l'on opère entre 40°C et 120°C ou entre le point de fusion du composé phénolique mis en oeuvre et 120°C.

16. Procédé selon la revendication 14, caractérisé en ce que le dichloro-2,6 phénol utilisé est obtenu par chloration à l'aide de chlore gazeux, de chloro-2 phénol, en présence d'une amine primaire, secondaire ou tertiaire telle que définie dans les revendications précédentes.

17. Procédé selon la revendication 14, caractérisé en ce que le dichloro-2,6 phénol utilisé est obtenu par chloration à l'aide de chlore gazeux, de phénol, en présence d'une amine primaire, secondaire ou tertiaire telle que définie dans les revendications précédentes.

18. Procédé selon l'une des revendications 1 à 17, caractérisé en ce qu'il est appliqué à des mélanges bruts de chloration du phénol, contenant du dichloro-2,6 phénol ainsi que du dichloro-2,4 phénol, de l'orthochlorophénol et éventuellement de faibles quantités de parachlorophénol et de phénol.

## Patentansprüche

1. Verfahren zur Chlorierung von Phenolverbindungen der allgemeinen Formel (I)

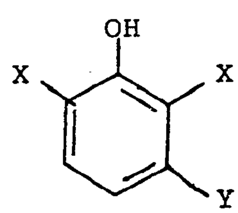

(I) ,

worin

— die Symbole X gleich oder verschieden sind und ein Chlor- oder ein Bromatom, eine Methyl- oder Ethylgruppe, eine Methoxy- oder Ethoxygruppe, eine Acetoxygruppe, eine $NO_2$-Gruppe und eine 1 bis 4 Kohlenstoffatome enthaltende Acylaminogruppe bedeuten und

— das Symbol Y ein Wasserstoffatom, eine Methyl- oder Ethylgruppe und eine Methoxy- oder Ethoxygruppe bedeutet,

durch Chlorgas in para-Stellung, bezogen auf die OH-Funktion, dadurch gekennzeichnet, daß man in Gegenwart einer wirksamen Menge wenigstens eines primären, sekundären oder tertiären Amins arbeitet.

2. Verfahren nach Anspruch 1, dadurch gekennzeichnet, daß man in der Schmelze arbeitet.

3. Verfahren nach Anspruch 1, dadurch gekennzeichnet, daß man in einem Lösungsmittel arbeitet.

4. Verfahren nach Anspruch 3, dadurch gekennzeichnet, daß das Lösungsmittel aus einem aliphatischen Ether, aliphatischen Kohlenwasserstoff, chlorierten aliphatischen Kohlenwasserstoff, einem Chlorbenzol oder einem Brombenzol besteht.

5. Verfahren nach einem der Ansprüche 1 bis 4, dadurch gekennzeichnet, daß man wenigstens ein Amin

verwendet, das ausgewählt ist aus den Aminen der allgemeinen Formel (II)

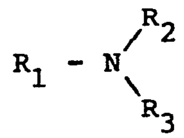 (II),

worin

— $R_1$, $R_2$ und $R_3$ gleich oder verschieden sind und

- einen 1 bis 12 Kohlenstoffatome enthaltenden geradkettigen Alkylrest, einen 3 bis 12 Kohlenstoffatome enthaltenden sekundären Alkylrest oder einen 4 bis 12 Kohlenstoffatome enthaltenden tertiären Alkylrest bedeuten, wobei diese Alkylreste eine oder zwei Ether- —O— Funktionen oder Hydroxyl-, Amin-, Carbonsäure-, Carbonsäureester-, Amid- oder Iminfunktionen enthalten können,
- einen Phenyl-, Cyclohexyl-, Cycloheptyl- oder Cyclopentylrest,
- einen Phenylalkyl-, Cyclohexylalkyl-, Cyloheptylalkyl- oder Cyclopentylalkylrest, dessen Alkylteil 1 bis 4 Kohlenstoffatome enthält, und
- ein Wasserstoffatom bedeuten,

— $R_1$ eine $NH_2$-Gruppe bedeuten kann,

— $R_2$ und $R_3$ zusammen und mit dem Stickstoffatom einen gesättigten oder eine oder mehrere Doppelbindungen enthaltenden Heterocyclus bilden, der gegebenenfalls durch eine oder mehrere 1 bis 4 Kohlenstoffatome enthaltende Alkylgruppen substituiert ist,

— $R_2$ und $R_3$ oder $R_1$, $R_2$ und $R_3$ zusammen, mit dem Stickstoffatom und mit einem oder mehreren Stickstoff- und/oder Sauerstoff- und/oder Schwefelatomen einen gesättigten oder ungesättigten Heterocyclus bilden, der gegebenenfalls durch eine oder mehrere 1 bis 4 Kohlenstoffatome enthaltende Alkylgruppen substituiert ist,

— $R_1$, $R_2$ und $R_3$ zusammen und mit dem Stickstoffatom einen ungesättigten Heterocyclus bilden, der gegebenenfalls durch eine oder zwei Methyl- oder Ethylgruppen substituiert ist, und

— $R_2$ und $R_3$ oder $R_1$, $R_2$ und $R_3$ zusammen, mit dem Stickstoffatom und gegebenenfalls mit einem oder mehreren Stickstoff- und/oder Sauerstoff- und/oder Schwefelatomen eine gesättigte oder ungesättigte polycyclische Verbindung bilden, die gegebenenfalls durch eine oder mehrere 1 bis 4 Kohlenstoffatome enthaltende Alkylgruppen substituiert ist.

6. Verfahren nach einem der Ansprüche 1 bis 5, dadurch gekennzeichnet, daß man 0,005 bis 25 Gew.% Amin, bezogen auf die Phenolverbindung der Formel (I), verwendet.

7. Verfahren nach Anspruch 2, dadurch gekennzeichnet, daß man 0,015 bis 5 Gew.% Amin, bezogen auf die Phenolverbindung (I), verwendet.

8. Verfahren nach Anspruch 3 oder 4, dadurch gekennzeichnet, daß man 0,01 bis 2 Gew.% Amin, bezogen auf das Reaktionsgemisch, verwendet.

9. Verfahren nach einem der Ansprüche 1 bis 8, dadurch gekennzeichnet, daß das verwendete Amin ausgewählt ist aus den primären oder sekundären Aminen der Formel (III)

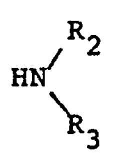 (III),

worin

— $R_2$ oder $R_3$ ein Wasserstoffatom bedeutet,

— $R_2$ und $R_3$ gleich oder verschieden sind und

- einen 1 bis 10 Kohlenstoffatome enthaltenden geradkettigen Alkylrest,
- einen 3 bis 10 Kohlenstoffatome enthaltenden sekundären Alkylrest,
- einen 4 bis 10 Kohlenstoffatome enthaltenden tertiären Alkylrest,
- einen Cyclohexyl- oder Cyclopentylrest,
- einen Phenylrest und einen Benzyl- oder Phenethylrest bedeuten,

— $R_2$ und $R_3$ zusammen, mit dem Stickstoffatom und mit einem anderen Stickstoff- und/oder Sauerstoffatom einen gesättigten oder eine oder mehrere ungesättigte Bindungen enthaltenden Heterocyclus bilden und

— $R_2$ und/oder $R_3$ eine oder mehrere Amin-, Hydroxyl- oder Carbonsäureesterfunktionen enthalten.

10. Verfahren nach einem der Ansprüche 1 bis 8, dadurch gekennzeichnet, daß das verwendete Amin aus den sekundären Aminen der allgemeinen Formel (III) ausgewählt ist, in der wenigstens eines der Symbole $R_2$ und $R_3$ und vorzugsweise beide Symbole $R_2$ und $R_3$

- einen solchen 3 bis 10 Kohlenstoffatome enthaltenden sekundären Alkylrest wie Isopropyl, 2-Butyl, 2-Pentyl, 3-Pentyl, 2-Hexyl, 3-Hexyl, 2-Heptyl, 3-Heptyl, 4-Heptyl, 2-Octyl, 3-Octyl, 4-Octyl, 2-Nonyl, 3-Nonyl, 4-Nonyl, 5-Nonyl, 2-Decyl, 3-Decyl, 4-Decyl und 5-Decyl und
- einen Cyclohexyl- oder Cyclopentylrest bedeuten, und denjenigen ausgewählt ist, in denen $R_2$ und $R_3$ zusammen mit dem Stickstoffatom einen Heterocyclus bilden, der gegebenenfalls ein anderes Stickstoff- oder ein Sauerstoffatom enthält.

11. Verfahren nach Anspruch 9 oder 10, dadurch gekennzeichnet, daß man Diisopropylamin, Diisobutylamin, Dicyclohexylamin, Morpholin und/oder Imidazol verwendet.

12. Verfahren nach Anspruch 9, dadurch gekennzeichnet, daß man n-Propylamin, Isopropylamin, n-Butylamin, Isobutylamin, tert.-Butylamin, n-Pentylamin, 2-Methylpentylamin, 3-Methylpentylamin, 2-Ethylhexylamin, Laurylamin, Cyclohexylamin, Cyclopentylamin, Benzylamin, Glycinethylester und/oder Ethanolamin verwendet.

13. Verfahren nach einem der Ansprüche 1 bis 12, dadurch gekennzeichnet, daß die Phenolverbindung der Formel (I) aus 2,6-Dichlorphenol, 2,6-Dimethoxyphenol, 2-Chlor-6-methoxyphenol, 2-Chlor-6-methylphenol, 2,6-Dichlor-3-methylphenol, 2,6-Dichlor-3-methoxyphenol, 2-Brom-6-methoxyphenol, 2-Chlor-6-nitrophenol und 2-Chlor-6-acetamidophenol ausgewählt ist.

14. Verfahren nach einem der Ansprüche 1 bis 12, dadurch gekennzeichnet, daß die Phenolverbindung der Formel (I) 2,6-Dichlorphenol ist.

15. Verfahren nach einem der Ansprüche 1 bis 14, dadurch gekennzeichnet, daß man zwischen 40 und 120°C oder zwischen dem Schmelzpunkt der eingesetzten Phenolverbindung und 120°C arbeitet.

16. Verfahren nach Anspruch 14, dadurch gekennzeichnet, daß das verwendete 2,6-Dichlorphenol durch Chlorierung von 2-Chlorphenol in Gegenwart eines solchen, wie in den vorhergehenden Ansprüchen definierten, primären, sekundären oder tertiären Amins mit Hilfe von Chlorgas erhalten wird.

17. Verfahren nach Anspruch 14, dadurch gekennzeichnet, daß das verwendete 2,6-Dichlorphenol durch Chlorierung von Phenol in Gegenwart eines solchen, wie in den vorhergehenden Ansprüchen definierten, primären, sekundären oder tertiären Amins mit Hilfe von Chlorgas erhalten wird.

18. Verfahren nach einem der Ansprüche 1 bis 17, dadurch gekennzeichnet, daß es auf Rohgemische der Phenolchlorierung angewendet wird, die 2,6-Dichlorphenol sowie 2,4-Dichlorphenol, o-Chlorphenol und gegebenenfalls geringe Mengen an p-Chlorphenol und an Phenol enthalten.

## Claims

1. Process for the chlorination, at the para position with respect to the OH group, with gaseous chlorine of phenolic compounds of general formula (I) :

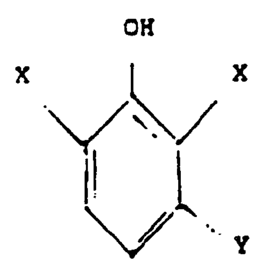

(I)

in which :

the symbols X, which may be identical or different, denote a chlorine atom, a bromine atom, a methyl or ethyl group, a methoxy or ethoxy group, an acetoxy group, an $NO_2$ group or an acylamino group having 1 to 4 carbon atoms,

the symbol Y denotes a hydrogen atom, a methyl or ethyl group or a methoxy or ethoxy group, characterised in that the reaction is performed in the presence of an effective quantity of at least one primary, secondary or tertiary amine.

2. Process according to claim 1, characterised in that the reaction is performed in the molten state.

3. Process according to claim 1, characterised in that the reaction is performed in a solvent.

4. Process according to claim 3, characterised in that the solvent consists of an aliphatic ether, an aliphatic hydrocarbon, a chlorinated aliphatic hydrocarbon, a chlorobenzene or a bromobenzene.

5. Process according to one of claims 1 to 4, characterised in that use is made of at least one amine chosen from the amines of general formula (II) :

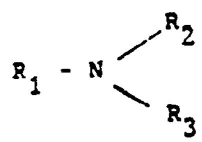 (II)

in which :

— $R_1$, $R_2$ and $R_3$, which may be identical or different, denote :

- a linear alkyl radical having from 1 to 12 carbon atoms, a secondary alkyl radical having 3 to 12 carbon atoms or a tertiary alkyl radical having 4 to 12 carbon atoms, it being possible for these alkyl radicals to contain one or two —O— ether groups or hydroxyl, amine, carboxylic acid, carboxylic acid ester, amide or imine groups ;
- a phenyl radical, a cyclohexyl radical, a cycloheptyl radical or a cyclopentyl radical ;
- a phenylalkyl, cyclohexylalkyl, cycloheptylalkyl or cyclopentylalkyl radical in which the alkyl portion contains 1 to 4 carbon atoms ;
- a hydrogen atom ;

— $R_1$ can denote an $NH_2$ group ;

— $R_2$ and $R_3$ form, together with the nitrogen atom, a heterocycle which is saturated or contains one or more double bonds, optionally substituted with one or more alkyl groups having 1 to 4 carbon atoms ;

— $R_2$ and $R_3$ or $R_1$, $R_2$ and $R_3$ form, together with the nitrogen atom and with one or more nitrogen and/or oxygen and/or sulphur atoms, a saturated or unsaturated heterocycle optionally substituted with one or more alkyl groups having 1 to 4 carbon atoms ;

— $R_1$, $R_2$ and $R_3$ form, together and with the nitrogen atom, an unsaturated heterocycle optionally substituted with one or two methyl or ethyl groups ;

— $R_2$ and $R_3$ or $R_1$, $R_2$ and $R_3$ form, together with the nitrogen atom and optionally with one or more nitrogen and/or oxygen and/or sulphur atoms, a saturated or unsaturated polycyclic compound optionally substituted with one or more alkyl groups having 1 to 4 carbon atoms.

6. Process according to one of claims 1 to 5, characterised in that from 0.005% to 25% by weight of amine is used relative to the phenolic compound of formula (I).

7. Process according to claim 2, characterised in that from 0.015% to 5% by weight of amine is used relative to the phenolic compound (I).

8. Process according to one of claims 3 and 4, characterised in that from 0.01% to 2% by weight of amine is used relative to the reaction mixture.

9. Process according to one of claims 1 to 8, characterised in that the amine used is chosen from the primary or secondary amines of formula (III) :

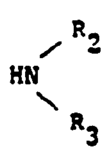 (III)

in which :

— $R_2$ or $R_3$ denotes a hydrogen atom ;

— $R_2$ and $R_3$, which may be identical or different, denote :

- a linear alkyl radical having 1 to 10 carbon atoms ;
- a secondary alkyl radical having 3 to 20 carbon atoms ;
- a tertiary alkyl radical having 4 to 10 carbon atoms ;
- a cyclohexyl or cyclopentyl radical ;
- a phenyl radical ;
- a benzyl or phenethyl radical ;

— $R_2$ and $R_3$, form, together with the nitrogen atom and with another nitrogen and/or oxygen atom, a saturated heterocycle or a heterocycle containing one or more unsaturated bonds ;
— $R_2$ and/or $R_3$, contain one or more amine, hydroxyl or carboxylic acid ester groups.

10. Process according to one of claims 1 to 8, characterised in that the amine used is chosen from the secondary amines of general formula (III) in which at least one of the symbols $R_2$ and $R_3$, and preferably both symbols $R_2$ and $R_3$, denote :

- a secondary alkyl radical having from 3 to 10 carbon atoms such as isopropyl, 2-butyl, 2-pentyl, 3-pentyl, 2-hexyl, 3-hexyl, 2-heptyl, 3-heptyl, 4-heptyl, 2-octyl, 3-octyl, 4-octyl, 2-nonyl, 3-nonyl, 4-nonyl, 5-nonyl, 2-decyl, 3-decyl, 4-decyl and 5-decyl ;
- a cyclohexyl or cyclopentyl radical ; and those for which $R_2$ and $R_3$ form, with the nitrogen atom, a heterocycle optionally containing another nitrogen atom or an oxygen atom.

11. Process according to one of claims 9 and 10, characterised in that diisopropylamine, diisobutylamine, dicyclohexylamine, morpholine and/or imidazole are/is used.

12. Process according to claim 9, characterised in that n-propylamine, isopropylamine, n-butylamine, isobutylamine, tert-butylamine, n-pentylamine, 2-methylpentylamine, 3-methylpentylamine, 2-ethylhexylamine, laurylamine, cyclohexylamine, cyclopentylamine, benzylamine, glycine ethyl ester and/or ethanolamine is/are used.

13. Process according to one of claims 1 to 12, characterised in that the phenolic compound of formula (I) is chosen from 2,6-dichlorophenol, 2,6-dimethoxyphenol, 2-chloro-6-methoxyphenol, 2-chloro-6-methylphenol, 2,6-dichloro-3-methylphenol, 2,6-dichloro-3-methoxyphenol, 2-bromo-6-methoxyphenol, 2-chloro-6-nitrophenol, and 2-chloro-6-acetamidophenol.

14. Process according to one of claims 1 to 12, characterised in that the phenolic compound of formula (I) is 2,6-dichlorophenol.

15. Process according to one of claims 1 to 14, characterised in that the reaction is performed at between 40°C and 120°C or between the melting point of the phenolic compound employed and 120°C.

16. Process according to claim 14, characterised in that the 2,6-dichlorophenol used is obtained by the chlorination of 2-chlorophenol using gaseous chlorine, in the presence of a primary, secondary or tertiary amine as defined in the preceding claims.

17. Process according to claim 14, characterised in that the 2,6-dichlorophenol used is obtained by the chlorinaton of phenol using gaseous chlorine, in the presence of a primary, secondary or tertiary amine as defined in the preceding claims.

18. Process according to one of claims 1 to 17, characterised in that it is applied to crude mixtures of chlorination of phenol, containing 2,6-dichlorophenol as well as 2,4-dichlorophenol, ortho-chlorophenol and possibly small amounts of para-chlorophenol and phenol.